# EUROPEAN PATENT APPLICATION

(11) **EP 1 637 038 A2**
(43) Date of publication of application: **22.03.2006**
(21) Application number: 05015095.2
(22) Date of filing: 12.07.2005
(51) Int. Cl.: A01N 57/08, C11D 1/66, C11D 3/30

(54) **N,N'-dialkyl derivatives of polyhydroxyalkyl alkylenediamines**

(30) Priority: 19.07.2004 US 894108
(71) Applicant: AIR PRODUCTS AND CHEMICALS, INC., Allentown, PA 18195-1501 (US)
(72) Inventor: Ford, Michael Edward, Trexlertown, PA 18087 (US); Kretz, Christine Peck, Macungie, PA 18062 (US); Lassila, Kevin Rodney, Westford, MA 01886 (US); Underwood, Richard Paul, Allentown, PA 18106 (US); Meier, Ingrid Kristine, Asbury, NJ 08802 (US)
(74) Representative: Schwabe - Sandmair - Marx

(57) **Abstract**

Surfactant compositions containing compounds according to structure (I), and methods of making them, are disclosed. The compounds provide reduced dynamic and equilibrium surface tension, good solubility, moderate foaming, and good cleaning performance. The methods for making them involve reaction of N-(polyhydroxyalkyl)-alkylamines with dinitriles, dialdehydes, or acetals or hemiacetals of dialdehydes in the presence of hydrogen and a transition metal catalyst. 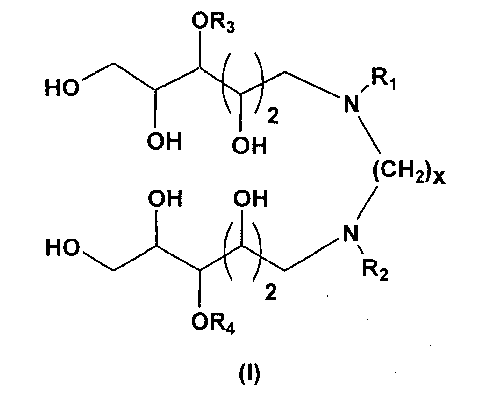 In structure (I), x is an integer from about 1 to 12, R₁ and R₂ are independently C3 - C30 linear alkyl, cyclic alkyl, branched alkyl, alkenyl, aryl, alkylaryl, alkoxyalkyl, and dialkylaminoalkyl; and R₃ and R₄ are independently hydrogen or a pyranosyl group such as α-D-glucopyranosyl, β-D-glucopyranosyl, or β-D-galactopyranosyl.

## Description

### FIELD OF THE INVENTION

This invention relates to N,N'-dialkyl-N,N'-bis(polyhydroxyalkyl)alkylenediamines, a new process for their manufacture, and their use to reduce the surface tension in water-based systems.

### BACKGROUND OF THE INVENTION

The ability to reduce the surface tension of water is of great importance in the application of water-based formulations because decreased surface tension translates to enhanced substrate wetting in during use. Examples of water-based compositions requiring good wetting include coatings, inks, adhesives, fountain solutions for lithographic printing, cleaning compositions, metalworking fluids, agricultural formulations, electronics cleaning and semiconductor processing compositions, personal care products, concrete admixtures, formulations for textile processing, and oilfield production and oil and gas recovery applications. Surface tension reduction in water-based systems is generally achieved through the addition of surfactants, resulting in enhanced surface coverage, fewer defects, and a more uniform distribution of the system. Equilibrium surface tension (EST) is important when the system is at rest, while dynamic surface tension (DST) provides a measure of the ability of a surfactant to reduce surface tension and provide wetting under high speed application conditions.

The importance of the ability of a surfactant to achieve low surface tension at low use levels, the ability to affect foaming performance, and the ability to provide efficient emulsification and solubilization are all of considerable industrial importance, as is well-appreciated in the art. And, although equilibrium surface tension reduction efficiency is important for some applications, other applications may require both equilibrium and dynamic surface tension reduction.

The foaming characteristics of a surfactant are also important because they can help define applications for which the surfactant might be suitable. For example, foam can be desirable for applications such as ore flotation, cleaning and personal care. On the other hand, in coatings, graphic arts and adhesive applications, foam is undesirable because it can complicate application and lead to defect formation. Thus foaming characteristics are frequently an important performance parameter.

The wide variety of applications for which surfactants are used, and the resultant variation in performance requirements, results in a need for a correspondingly large number of surfactants adapted to these various performance demands, and a need for suitable methods for making them.

### SUMMARY OF THE INVENTION

In one aspect, the invention is a composition including at least one compound according to structure (I): 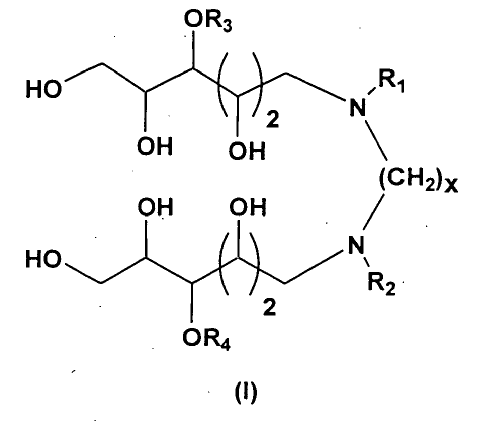 wherein x is an integer from 1 to 12; R₁ is 1-octyl and R₂ is 1-octyl or 1-butyl; and R₃ and R₄ are independently selected from the group consisting of hydrogen, α-D-glucopyranosyl, β-D-glucopyranosyl, and β-D-galactopyranosyl.

In another aspect, the invention is a composition including a compound according to structure 1 above, wherein x is an integer from 2 to 6; R₁ is methyl or ethyl and R₂ is 1-hexyl or 1-octyl; and R₃ and R₄ are both H.

In yet another aspect, the invention is a composition including a compound according to structure 1 above, wherein x is an integer from 2 to 6; R₁ is (CH2)2OCH3 or (CH2)3OCH3 and R₂ is 1-hexyl or 1-octyl; and R₃ and R₄ are both H.

In still another aspect, the invention is a method of making a compound according to structure (I) above, wherein x is an integer from 1 to 12; R₁ and R₂ are independently selected from the group consisting of C3 - C30 linear alkyl, cyclic alkyl, branched alkyl, alkenyl, aryl, alkylaryl, alkoxyalkyl, and dialkylaminoalkyl; and R₃ and R₄ are independently selected from the group consisting of hydrogen, α-D-glucopyranosyl, β-D-glucopyranosyl, and β-D-galactopyranosyl. The method includes contacting an N-(polyhydroxyalkyl)alkylamine with a dinitrile, a dialdehyde, or an acetal or hemiacetal thereof. The contacting is performed in the presence of hydrogen and a transition metal catalyst.

### DETAILED DESCRIPTION OF THE INVENTION

### N,N'-dialkyl-N,N'-bis(polyhydroxyalkyl)alkylenediamines

Compositions according to the invention include at least one N,N'-dialkyl-N,N'-bis(polyhydroxyalkyl)alkylenediamine according to the following structure (I):

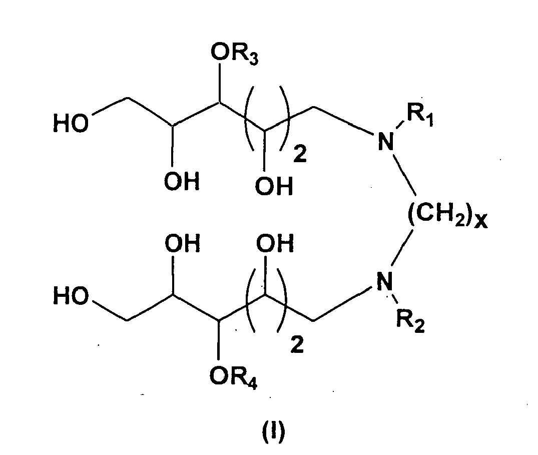

In structure (I), x is an integer from 1 to 12, typically from 2 to 4, R₁ and R₂ are independently selected from the group consisting of C3 - C30 linear alkyl, cyclic alkyl, branched alkyl, alkenyl, aryl, alkylaryl, alkoxyalkyl, and dialkylaminoalkyl; and R₃ and R₄ are independently selected from the group consisting of hydrogen, α-D-glucopyranosyl, β-D-glucopyranosyl, and β-D-galactopyranosyl.

Although any of a variety of polyhydroxyalkyl groups may be incorporated in compounds according to the invention, they most typically will be derived from the open-chain forms of reducing sugars, for example glucose. Therefore, for simplicity of explanation, these compounds are exemplified herein as glucose derivatives such as may be obtained by the reaction of an N-(1-deoxyglucityl)alkylamine with glyoxal, combined with a reduction employing a transition metal catalyst and hydrogen, as will be discussed below. Thus, exemplary glucose-derived compounds made according to the invention have the following structure, wherein R₁, R₂, and x are as defined above in relation to structure (I), 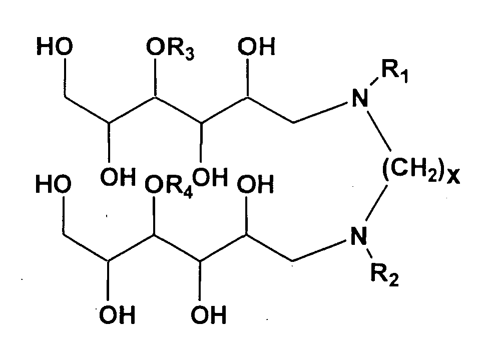 and R₃ and R₄ are both hydrogen.

The N-(polyhydroxyalkyl)alkylamine with which glyoxal or another dialdehyde is reacted can be prepared by reductive amination of a polyhydroxyalkyl compound, such as a glucose or other suitable mono- or disaccharide, with the desired amine. One way of doing this is shown in U.S. Pat. No. 5,449,770, Example 1, where glucose reacts with R₁-NH₂ (where R₁ may for example be methyl) to give an N-(polyhydroxyalkyl)alkylamine according to the following structure, where R₃ is H.

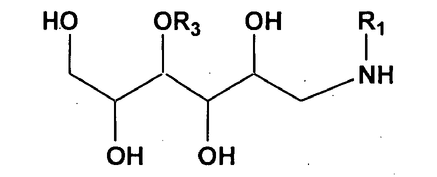

The corresponding compound with R₂ instead of R₁ and R₄ instead of R₃ may be made in an analogous way. In general, the polyhydroxyalkyl groups of N-(polyhydroxyalkyl)alkylamines useful for making N,N'-dialkyl-N,N'-bis(polyhydroxyalkyl)alkylenediamines according to the invention may be derived from any of the group of reducing sugars consisting of glucose, fructose, maltose, lactose, galactose, mannose, and xylose. Typically, the reducing sugar will be an aldose, although ketoses may also be used, and both monosaccharides and disaccharides may be used, with convenient sources of the latter including high dextrose corn syrup, high fructose corn syrup, and high maltose corn syrup. Other useful polyhydroxyalkyl groups may be derived from glyceraldehydes. In one embodiment, the polyhydroxyalkyl group is derived from glucose; i.e. the group is 1-deoxyglucityl.

The alkylamine with which the reducing sugar or other polyhydroxyalkyl group precursor is reacted may be represented by the formula below.

**R―NH**_{**2**}

The group R may be a linear, cyclic, or branched alkyl, alkoxyalkyl, dialkylaminoalkyl, alkenyl, aryl, or alkylaryl group having from 3 to about 30 carbon atoms, typically from about 4 to about 18 carbon atoms, and more typically from about 4 to about 14 carbon atoms. Examples of primary amines include, but are not limited to, propylamine, isopropylamine, n-butylamine, isobutylamine, n-pentylamine, isopentylamine, cyclopentylamine, n-hexylamine, cyclohexylamine, n-heptylamine, n-octylamine, 2-ethylhexylamine, isooctylamine, n-decylamine, n-dodecylamine, 3-methoxypropylamine, 3-ethoxypropylamine, 3-n-propoxypropylamine, 3-isopropoxypropylamine, 3-n-hexyloxypropylamine, 3-isohexyloxypropylamine, 3-[(2-ethyl)hexyloxy]propylamine, 3-isodecyloxypropylamine, 3-isotridecyloxypropylamine, 3-dodecyloxypropylamine, 3-isododecyloxypropylamine, 3-tetradecyloxypropylamine, mixed octyloxy-decyloxypropylamines (Tomah PA-1214, available from Tomah Products, Inc. of Milton, WI), mixed tetradecyloxydodecyloxypropylamines (Tomah PA-1816), mixed dodecyloxy-tetradecyloxypropylamines (Tomah PA-1618), mixed dodecyloxy-pentadecyloxypropylamines (Tomah PA-19), mixed octadecyloxy-hexadecyloxypropylamines (Tomah PA-2220), 3-dimethylaminopropylamine, 3-diethylaminopropylamine, 3-di-n-hexylpropylamine, stearylamine, and mixtures of amines derived from natural sources such as cocoalkylamine, oleylamine, and tallowamine. More preferred amines are butylamine, n-hexylamine, n-octylamine, and decylamine. As used herein, the meaning of "alkylamine" as used in the terms "N-(1-deoxyglucityl)alkylamine" and "N-(polyhydroxyalkyl)alkylamine" is to be understood to include both simple and substituted alkylamines, non-limiting examples of which are set forth in the foregoing part of this paragraph.

The linking group (CH₂)ₓ for compounds (I) may vary from x = 1 to x = 12. One particularly suitable linking group is.a two-carbon unit (x = 2). It is most typically obtained by reaction of the desired N-(polyhydroxyalkyl)alkylamine(s) with glyoxal, although functional equivalents may also be used, as will be discussed below. A three-carbon linking group may be obtained by reaction of desired N-(polyhydroxyalkyl)alkylamine(s) with malonaldehyde, while reaction of the desired N-(polyhydroxyalkyl)alkylamine(s) with 2,5-dimethoxytetrahydrofuran provides compounds (I) with a four-carbon linking group (x = 4).

Exemplary compounds (I) of the present invention are N,N'-dibutyl-N,N'-bis(1-deoxyglucityl)-1,2-diaminoethane; N,N'-dihexyl-N,N'-bis(1-deoxyglucityl)-1,2-diaminoethane; N,N'-dioctyl-N,N'-bis(1-deoxyglucityl)-1,2-diaminoethane; and N-butyl-N'-octyl-N,N'-bis(1-deoxyglucityl)-1,2-diaminoethane. Other exemplary surfactants according to the invention are mixtures of compounds (I), for example mixtures of N,N'-dibutyl-N,N'-bis(1-deoxyglucityl)-1,2-diaminoethane, N,N'-dioctyl-N,N'-bis(1-deoxyglucityl)-1,2-diaminoethane; and N-butyl-N'-octyl-N,N'-bis(1-deoxyglucityl)-1,2-diaminoethane, particularly an approximately 1:1:2 molar mixture of these, respectively. Such a mixture may be prepared by the reaction of equimolar amounts of N-octyl-D-glucamine and N-butyl-D-glucamine with glyoxal, by methods that will now be described.

### Preparation of the N,N'-dialkyl-N,N'-bis(polyhydroxyalkyl)alkylenediamines

In one exemplary preparative procedure, compounds according to the invention may be prepared by the reaction of an N-(polyhydroxyalkyl)alkylamine with a dialdehyde, typically in the presence of a solvent, at a temperature sufficiently high so as to provide a convenient reaction rate and sufficiently low so as to prevent significant by-product formation. The reaction temperatures may be in the range from about 50°C to about 175°C, typically from about 50°C to about 150°C, and more typically from about 60°C to about 125°C. The optimum conditions will depend upon the reactor configuration, the solvents employed, and other variables. The N-(polyhydroxyalkyl)alkylamines may be prepared using procedures such as those described in US 5,449,770 to Shumate et al.

The linking of the N-(polyhydroxyalkyl)alkylamine(s) with a dialdehyde to form the corresponding compound (I) requires the presence of a catalyst and hydrogen. The catalyst is typically a metal chosen from the group consisting of iron, cobalt, nickel, ruthenium, rhodium, palladium, osmium, iridium or platinum. Typically, the catalyst is selected from the group consisting of ruthenium, rhodium, palladium, or platinum, and more typically is either palladium or platinum. To maximize productivity, the catalyst is typically dispersed on a support. Such a support may be organic, such as carbon, or inorganic. Examples of the latter class of supports include alumina, silica, titania, magnesia, zirconia, and aluminosilicates. The preferred support for the catalyst is carbon.

The hydrogen pressure may be in the range from about 250 psig to 1500 psig, typically from about 500 psig to about 1250 psig, and more typically from about 750 psig to about 1100 psig. A variety of solvents may be used for the reaction. Examples of suitable solvents and solvent mixtures include, but may not be limited to, methanol, ethanol, ethylene glycol, propylene glycol, water/methanol, water/ethanol and mixtures thereof. The most preferred solvent combination is water/methanol.

Functional equivalents of dialdehydes, such as bis(dimethyl acetal) compounds or dinitriles, may also be used. Thus, for example, glyoxal bis(dimethyl acetal) (or other acetal, such as diethyl, etc), or a corresponding hemiacetal, or cyanogen may provide 2-carbon linking groups, while 3-carbon links may be provided by malonaldehyde, the corresponding bis(dimethyl acetal), or malononitrile.

### Surfactant Performance

The performance properties of compounds according to structure (I) may be optimized for a specific application by appropriate modification of the structure of the pendant polyhydroxyalkyl group, the diamine chain length x, and the choice of the substituents R₁ and R₂ on the diamine. The interplay among these factors is complex, and thus a certain amount of routine experimentation may be required to find optimal combinations of these variables for particular applications. These compounds may be useful as emulsifiers or detergents, wetting agents, foaming agents, defoamers, rheology modifiers or associative thickeners, dispersants, and the like. As such, they may for example find use in applications such as coatings, inks, adhesives, agricultural formulations, fountain solutions, photoresist strippers/developers, templating agents for mesoporous materials, soaps, shampoos, other cleaning compositions, and in cement admixture formulations. The compounds may also find use in oil-field applications such as enhanced oil recovery, fracturing and stimulation processes; and drilling and cementing operations, and in various wet-processing textile operations, such as dyeing of fibers and fiber scouring and kier boiling.

The term "water-based", "waterborne", "aqueous", or "aqueous medium", as used herein, means a solvent or liquid dispersing medium which includes water, typically at least 10 wt%, more typically 50 wt%, and most typically at least 95 wt%, water. The medium may be essentially only water.

### Uses of N,N'-Dialkyl-N,N'-bis(polyhydroxyalkyl)alkylenediamines

The invention further provides aqueous compositions including an effective amount of a surfactant according to structure (I) disclosed above, which compositions provide superior wetting. Suitable effective amounts for providing good wetting in water-based compositions, which may contain organic and/or inorganic species, may range from 0.001 wt% to 45 wt%, typically from 0.005 wt% to 25 wt%, and most typically from 0.01 wt% to 10 wt%, based on total weight of the formulation. The most favorable amount will vary from one application to another, depending upon the foam and wetting contributing species in that system. Exemplary non-limiting uses of the compounds of structure (I) according to the invention will now be outlined.

A typical water-based coating formulation that includes the surfactants of the invention may include the following components in an aqueous medium at 30 to 80% solids:

**Typical Aqueous-Based Coating Formulation**

| | |
|---|---|
| 0 to 50 wt% | Pigment Dispersant/Grind Resin |
| 0 to 80 wt% | Coloring Pigments/Extender Pigments/Anti-Corrosive Pigments/Other Pigment Types |
| 5 to 99.9 wt% | Water-Borne/Water-Dispersible/Water-Soluble Resins |
| 0 to 30 wt% | Slip Additives/Antimicrobials/Processing Aids/Defoamers |
| 0 to 50 wt% | Coalescing or Other Solvents |
| 0.01 to 10 wt% | Surfactant/Wetting/Flow and Leveling Agents, other than N,N'-dialkyl-N,N'-bis(polyhydroxyalkyl)alkylenediamines |
| 0.001 to 5 wt% | N,N'-dialkyl-N,N'-bis(polyhydroxyalkyl)alkylenediamine(s) |

A typical water-based ink composition that includes the surfactants of the invention may include the following components in an aqueous medium at 20 to 60% solids:

**Typical Aqueous-Based Ink Composition**

| | |
|---|---|
| 1-50 wt% | Pigment |
| 0 to 50 wt% | Pigment Dispersant/Grind Resin |
| 0 to 50 wt% | Clay base in appropriate resin solution vehicle |
| | Water-borne/water-dispersible/water-soluble |
| 5 to 99.9 wt% | resins |
| 0 to 30 wt% | Coalescing Solvents |
| 0.01 to 10 wt% | Surfactant/Wetting Agents, other than N,N'-dialkyl-N,N'-bis(polyhydroxyalkyl)alkylenediamines |
| 0.01 to 10 wt% | Processing Aids/Defoamers/Solubilizing Agents |
| 0.001 to 5 wt% | N,N'-dialkyl-N,N'-bis(polyhydroxyalkyl)alkylenediamine(s) |

A typical water-based agricultural composition that includes the surfactants of the invention may include the following components in an aqueous medium at 0.01 to 80% of the following ingredients:

**Typical Aqueous-Based Agricultural Composition**

| | |
|---|---|
| 0.1-50 wt% | Pesticide or Plant Growth Modifying Agent |
| 0.01 to 10 wt% | Surfactants, other than N,N'-dialkyl-N,N'-bis(polyhydroxyalkyl)alkylenediamines |
| 0 to 5 wt% | Dyes |
| 0 to 20 wt% | Thickeners/Stabilizers/Co-surfactants/Gel In-hibitors/Defoamers |
| 0 to 25 wt% | Antifreeze agent (e.g. ethylene glycol or propylene glycol) |
| 0.001 to 5 wt% | N,N'-dialkyl-N,N'-bis(polyhydroxyalkyl)alkylenediamine(s) |

A typical fountain solution composition for planographic printing that includes the surfactants of the invention may include the following components:

**Typical Fountain Solution for Planographic Printing**

| | |
|---|---|
| 0.05 to 10 wt% | Film forming, water soluble macromolecule |
| 1 to 25 wt% | Alcohol, glycol, or polyol with 2-12 carbon atoms, water soluble or can be made to be water soluble |
| | Water soluble organic acid, inorganic acid, or |
| 0.01 to 20 wt% | a salt of these |
| 30 to 98.939 wt% | Water |
| 0.001 to 5 wt% | N,N'-dialkyl-N,N'-bis(polyhydroxyalkyl)alkylenediamine(s) |

A typical hard surface cleaner that includes the surfactants of the invention may include the following components:

**Typical Hard Surface Cleaner**

| | |
|---|---|
| 0 to 25 wt% * | Anionic surfactant |
| 0 to 25 wt% * | Cationic surfactant |
| 0 to 25 wt% * | Nonionic surfactant (e.g. alcohol alkoxylates, etc.) |
| 0 to 20 wt% | Chelating agent (EDTA, citrate, tartrate, etc.) |
| 0 to 20 wt% * | Solvent (Glycol ether, lower alcohols, etc.) |
| 0.001 to 25 wt% | N,N'-dialkyl-N,N'-bis(polyhydroxyalkyl)alkylenediamine(s) |
| 0 to 2 wt% | Dye, fragrance, preservative, etc. |
| 0 to 40 wt% * | Alkali metal hydroxide |
| Balance to 100 wt% | Water, and optionally other ingredients |

| | |
|---|---|
| * To total, in combination, 0.1 to 99 wt%. | |

A typical water-based photoresist developer or electronic cleaning composition that includes the surfactants of the invention may include the following components:

**Typical Aqueous-Based Photoresist Developer Composition**

| | |
|---|---|
| 0.1 to 3 wt% | Tetramethylammonium hydroxide |
| 0 to 4 wt% | Phenolic resin |
| 92.5 to 99.9 wt% | Water |
| 0.001 to 5 wt% | N, N'-dialkyl-N, N'-bis(polyhydroxyalkyl)alkylenediamine(s) |

A typical metalworking fluid that includes the surfactants of the invention may include the following components:

**Typical Synthetic Metalworking Fluid Formulation**

| | |
|---|---|
| 2.5 to 10 wt% | Block copolymer or other emulsifying agent |
| 10 to 25 wt% | Alkanolamine |
| 2 to 10 wt% | Organic monoacid |
| 0 to 5 wt% | Organic diacid |
| 40 to 84.499 wt% | Water |
| 1 to 5 wt% | Biocide |
| 0.001 to 5 wt% | N,N'-dialkyl-N,N'-bis(polyhydroxyalkyl)alkylenediamine(s) |

Many different surfactant-containing formulations are used in products within the Personal Care Products and Household and Industrial & Institutional Cleaning markets. Surfactants according to the invention may be used in any of these formulations to provide one or more benefits, with the specific use of the surfactant depending upon the its structure. Typical formulations used in these markets are described in Louis Ho Tan Tai's book, *Formulating Detergents and Personal Care Products: A Complete Guide to Product Development* (Champaign, IL: AOCS Press, 2000) as well as in other books, literature, product formularies, etc. familiar to those skilled in the art. A few representative example formulations are described here as illustrations. For example, a rinse aid for use in household automatic dishwashing or in industrial and institutional warewashing may have the ingredients described below.

**Typical Rinse Aid Formulation**

| | |
|---|---|
| N,N'-dialkyl-N,N'-bis(polyhydroxyalkyl)alkylenediamine(s) | 0.001 to 45 wt% |
| Nonionic surfactant other than an N,N'-dialkyl-N,N'-bis(polyhydroxyalkyl)alkylenediamine (e.g. alkoxylated alcohol(s), alkoxylated block copolymers, etc.) | 0 to 45 wt% |
| Hydrotrope (e.g. sodium xylenesulfonate, sodium toluenesulfonate, anionic surfactant(s), amphoteric surfactant(s), etc.) | 0 to 10 wt% |
| Isopropyl alcohol or ethyl alcohol | 0 to 10 wt% |
| Chelant (e.g. citric acid, etc.) | 5 to 20 wt% |
| Water, and optionally other ingredients | Balance to 100 wt% |

**Typical Powdered Laundry Detergent Formulation**

| Material | Amount by Weight in Conventional Formulation | Amount by Weight in Concentrated Formulation |
|---|---|---|
| N,N'-dialkyl-N,N'-bis(polyhydroxyalkyl)alkylenediamine(s) | 0.001 to 5 wt% | 0.001 to 15 wt% |
| Detergent surfactant(s) (e.g. anionic surfactants, alcohol alkoxylates, etc.) | 0.1 to 30 wt% | 0.1 to 50 wt% |
| Builder/co-builder (zeolites, sodium carbonate, phosphates, etc.) | 25 to 50 wt% | 25 to 60 wt% |
| Bleach and bleach activator (perborates, etc.) | 0 to 25 wt% | 0 to 25 wt% |
| Other Additives (fragrance, enzymes, hydrotropes, etc.) | 0 to 7 wt% | 1 to 10 wt% |
| Fillers (sodium sulfate, etc.) | 5 to 35 wt% | 0 to 12 wt% |

**Typical Aqueous Liquid Laundry Detergent Formulation**

| Material | Amount by Weight in Conventional Formulation | Amount by Weight in Concentrated Formulation |
|---|---|---|
| N,N'-dialkyl-N,N'-bis(polyhydroxyalkyl)alkylenediamine(s) | 0.001 to 25 wt% | 0.001 to 30 wt% |
| Detergent surfactant(s) (e.g. anionic surfactants, alcohol alkoxylates, etc.) | 0 to 35 wt% | 0 to 65 wt% |
| Builder/co-builder (citrate, tartrate, etc.) | 3 to 30 wt% | 0 to 36 wt% |
| Other Additives (fragrances, dyes, etc.) | 0.1 to 5 wt% | 1 to 5 wt% |
| Water and other solvents (e.g. lower alcohols) | 5 to 75 wt% | 1 to 56 wt% |

**Typical Non-Aqueous Laundry Detergent Formulation**

| Material | Amount by Weight |
|---|---|
| N,N'-dialkyl-N,N'-bis(polyhydroxyalkyl)alkylenediamine(s) | 0.001 to 30 wt% |
| Detergent surfactant(s) (e.g. anionic surfactants, alcohol alkoxylates, amine oxides, etc.) | 0.1 to 42 wt% |
| Builder/co-builder (zeolites, sodium carbonate, phosphates, citrate or tartrate salts, etc.) | 25 to 60 wt% |
| Bleach and bleach activator (perborates, etc.) | 0 to 20 wt% |
| Anti-redeposition aids (sodium carboxymethylcellulose, etc.) | 0.5 to 5 wt% |
| Other Additives (fragrance, enzymes, etc.) | 0 to 5 wt% |
| Polyalkylene glycol | 0 to 50 wt% |

**Typical 2-Part Industrial and Institutional Laundry Formulation**

| | Amount by Weight of Material in Each Pack |
|---|---|
| **Pack A** | |
| N,N'-dialkyl-N,N'-bis(polyhydroxyalkyl)alkylenediamine(s) | 0.001 to 20 wt% |
| Detergent surfactant(s) (e.g. anionic surfactants, alcohol alkoxylates, etc.) | 0 to 20 wt% |
| Antiredeposition aids (sodium carboxymethylcellulose, etc.) | 0.01 to 2 wt% |
| Water, and optionally other ingredients | Balance to 100 wt% |

| **Pack B** | |
|---|---|
| Sodium silicate | 5 to 10 wt% |
| Sodium metasilicate | 0 to 30 wt% |
| Tetrapotassium pyrophosphate | 0 to 10 wt% |
| potassium hydroxide | 0 to 35 wt% |
| potassium carbonate | 0 to 15 wt% |
| Water, and optionally other ingredients | Balance to 100 wt% |
| **Mix Ratio Pack A:Pack B** | 1:2 to 1:4 |

**Typical Shampoo or Liquid Body Wash Formulation**

| Material | Amount by Weight |
|---|---|
| N,N'-dialkyl-N,N'-bis(polyhydroxyalkyl)alkylenediamine(s) | 0.001 to 5 wt% |
| Anionic surfactant(s) (e.g. sodium or ammonium lauryl sulfate, sodium or ammonium lauryl sulfate, etc.) | 0.1 to 30 wt% |
| Amphoteric cosurfactant(s) (e.g. cocoamidopropyl betaine, etc.) | 0 to 20 wt% |
| Nonionic surfactant other than an N,N'-dialkyl-N,N'-bis(polyhydroxyalkyl)alkylenediamine (e.g. alcohol alkoxylates, sorbitan esters, alkyl glucosides, etc.) | 0 to 20 wt% |
| Cationic polymers (e.g. polyquaternium, etc.) | 0 to 5 wt% |
| Other Additives (fragrance, dyes, oils, opacifiers, preservatives, chelants, hydrotropes, etc.) | 0 to 15 wt% |
| Polymeric thickeners (e.g. polyacrylate, etc.) | 0 to 2 wt% |
| Conditioning oils (e.g. sunflower oil, petrolatum, etc.) | 0 to 10 wt% |
| Citric acid | 0 to 2 wt% |
| Ammonium chloride or sodium chloride | 0 to 3 wt% |
| Humectants (e.g. propylene glycol, glycerin, etc.) | 0 to 15 wt% |
| Glycol distearate | 0 to 5 wt% |
| Cocoamide (i.e. cocoamide MEA, cocoamide MIPA, PEG-5 cocoamide, etc.) | 0 to 10 wt% |
| Dimethicone | 0 to 5 wt% |
| Behenyl alcohol | 0 to 5 wt% |
| Water, and optionally other ingredients | Balance to 100 wt% |

**Typical Hair Conditioner Formulation**

| Material | Amount by Weight |
|---|---|
| N,N'-dialkyl-N,N'-bis(polyhydroxyalkyl)alkylenediamine(s) | 0.001 to 10 wt% |
| Nonionic surfactant other than an N,N'-dialkyl-N,N'-bis(polyhydroxyalkyl)alkylenediamine, and/or fatty alcohol(s) (e.g. stearyl alcohol, etc.) | 0.1 to 10 wt% |
| Cationic surfactant(s) (e.g. cetrimonium chloride, etc.) | 0 to 10 wt% |
| Anionic surfactants (e.g. TEA-dodecylbenzenesulfonate, etc.) | 0 to 5 wt% |
| Silicones (e.g. dimethicone, dimethiconal, etc.) | 0 to 5 wt% |
| Cationic polymers (e.g. polyquaternium, etc.) | 0 to 10 wt% |
| Other Additives (fragrance, dyes, oils, opacifiers, preservatives, chelants, hydrotropes, etc.) | 0 to 10 wt% |
| Thickening polymers (e.g. hydroxyethylcellulose, polyacrylates, etc.) | 0 to 5 wt% |
| Potassium, ammonium or sodium chloride | 0 to 5 wt% |
| Humectant (e.g. propylene glycol, etc.) | 0 to 5 wt% |
| Panthenol | 0 to 2 wt% |
| Water, and optionally other ingredients | Balance to 100 wt% |

**Typical Aqueous Sunscreen Formulation**

| Material | Amount by Weight |
|---|---|
| N,N'-dialkyl-N,N'-bis(polyhydroxyalkyl)alkylenediamine(s) | 0.001 to 30 wt% |
| Polyethylene glycol (e.g. PEG-8, etc.) | 0 to 30 wt% |
| Active sunscreen agents (e.g. octyl methoxycinnamate, azobenzone, homosalate, octyl salicylate, oxybenzone, octocrylene, butyl methoxydibenzoylmethane, octyl triazone, etc.) | 1 to 30 wt% |
| Esters and emollients (e.g. dimethicone, methylparaben, propylparaben, polysorbates, etc.) | 0 to 20 wt% |
| Thickening polymers (e.g. acrylates/C10-30 alkyl acrylate crosspolymer, PVP/hexadecene copolymer, etc.) | 0 to 20 wt% |
| Other Additives (fragrance, dyes, oils, opacifiers, preservatives, chelants, etc.) | 0 to 15 wt% |
| Solvent/hydrotropes (e.g. propylene glycol, benzyl alcohol, dicapryl ether, etc.) | 0 to 20 wt% |
| Triethanolamine | 0 to 5 wt% |
| Water, and optionally other ingredients | Balance to 100 wt% |

### Cement Admixture Formulations

Cement admixtures may be of any of several types, including superplasticizing, plasticizing, accelerating, set retarding, air entraining, water-resisting, corrosion inhibiting, and other types. Such admixtures are used to control the workability, settling and end properties (strength, impermeability, durability and frost/deicing salt resistance, etc.) of cementitious products like concretes, mortars, etc. The admixtures are usually provided as aqueous solutions and they can be added to the cementitious system at some point during its formulation. Surfactants of this invention may provide wetting, foam control, flow and levelling, water reduction, corrosion inhibition, high ionic strength tolerance and compatibility, and other benefits when used in such systems.

**Exemplary Cement Admixture Ingredients**

| Material | Amount by Weight Relative to Cement Weight |
|---|---|
| N,N'-dialkyl-N,N'-bis(polyhydroxyalkyl)alkylenediamine(s) | 0.001 to 5 wt% |
| Solubilizing agents (solvent, hydrotropes, amines, etc.) * | 0 to 10 wt% |
| Polymers and/or oligomers (e.g. lignosulfonates, sulfonated melamine formaldehyde condensates, polycarboxylates, styrene-maleic anhydride oligomers, copolymers and their derivatives, etc.) * | 0 to 5 wt% |
| Functional Additives (defoamers, air entraining or detraining agents, pH control additives, corrosion inhibitors, set retarders, accelerators, preservatives, etc.) * | 0 to 5 wt% |
| Water | 40 to 75% |

| | |
|---|---|
| * To total, in combination, 0.1 to 20 wt%. | |

The present invention is further illustrated by the following examples, which are presented for purposes of demonstrating, but not limiting, the methods and compositions of this invention.

### EXAMPLES

Examples 1-7 illustrate one particularly suitable process for preparing compounds according to the invention, via coupling of a1-deoxy-1-(alkylamino)-D-glucitol or mixture of 1-deoxy-1-(alkylamino)-D-glucitols with glyoxal in the presence of a catalyst at elevated temperature and pressure of hydrogen. This transformation is represented by the following equation: 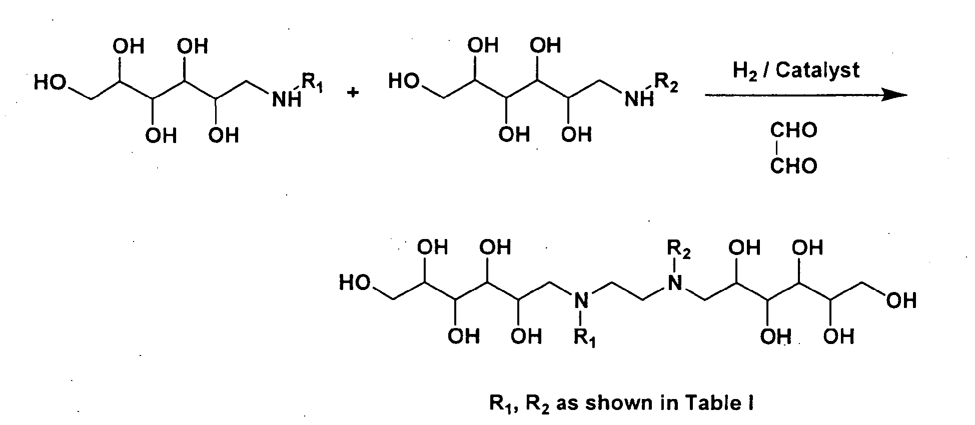 The preparation of N,N'-dioctyl-N,N'-bis(1-deoxyglucityl)ethylenediamine is used for illustration.

### Examples 1 - 7.

A 100 mL Parr stainless steel reactor was charged with 2.93 gm (0.01 mole) 1-deoxy-1-(octylamino)-D-glucitol, 0.70 gm of 40% aqueous glyoxal (0.00483 mole; 0.966 equivalent), 0.14 gm (dry weight basis) 5% palladium on carbon, and 30 gm of methanol. The reactor was closed, purged with nitrogen and hydrogen, and pressurized to ca 600 psig with hydrogen. The mixture was heated with stirring (1000 rpm) to 125°C and pressurized with hydrogen to 1000 psig. The reaction was maintained at this temperature; pressure was maintained at 1000 psig via regulated hydrogen feed. After 12 hr, the mixture was cooled to room temperature, and the product removed from the reactor by filtration through an internal 0.5µ sintered metal element. After trimethylsilylation, analysis of the product by gas chromatography (GC) and gas chromatography/mass spectroscopy (GC-MS) indicated that it consisted of 85% N,N'-dioctyl-N,N'-bis(1-deoxyglucityl)-ethylenediamine, 6% N-octyl-N-(2-hydroxyethyl)-1-deoxyglucitylamine, 2% unchanged 1-deoxy-1-(octylamino)-D-glucitol, and minor amounts of byproducts. Additional N,N'-bis(1-deoxyglucityl)alkylenediamines were prepared and characterized using procedures similar to that above. Some of the N,N'-bis(1-deoxyglucityl) alkylenediamines that were prepared and their designations are shown in Table 1.

### Example 8.

This example illustrates another aspect of this invention, the ability to use sugars other than α-D-glucose to prepare surfactants with carbohydrate groups other than 1-deoxyglucityl.

The procedure of examples 1 - 7 was followed, with substitution of 4.55 gm (0.01 mole) 1-deoxy-1-(aminooctyl)-D-maltitol in place of the 1-deoxy-1-(aminoalkyl)glucitol used previously. Owing to the high molecular weight of the resulting gemini surfactant, analysis by GC or GC-MS after trimethylsilylation was unsuccessful. However, analysis by MALD/I MS showed that the desired N,N'-dioctyl-N,N'-bis(deoxymaltitol)-ethylenediamine had been formed, along with a lesser amount of N-octyl-N-(2-hydroxyethyl)-1-deoxymaltitol.

**Table 1. N,N'-Dialkyl-N,N'-Bis(1-deoxyglucityl)alkylenediamines**

| Example | NB Reference | Spacer Length (x) | R₁ | R₂ | Abbreviation |
|---|---|---|---|---|---|
| 1 | 18593-85 | 2 | n-C₈H₁₇ | n-C₈H₁₇ | DODGEDA |
| 2 | 18838-34 | 2 | n-C₆H₁₃ | n-C₆H₁₃ | DHDGEDA |
| 3 | 18838-35 | 2 | n-C₄H₉ | n-C₄H₉ | DBDGEDA |
| 4 | 18588-47^{a} | 2 | n-C₈H₁₇ | n-C₄H₉ | BODGEDA |
| 5 | 18588-46 | 2 | CH₃O(CH₂)₃ | CH₃O(CH₂)₃ | MPDGEDA |
| 6 | 18588-73 | 2 | n-C₄H₉O(CH₂)₃ | n-C₄H₉O(CH₂)₃ | BPDGEDA |
| 7 | 18588-73 | 2 | cyclo-C₆H₁₃ | cyclo-C₆H₁₃ | DCHDGEDA |

| | | | | | |
|---|---|---|---|---|---|
| Note to Table 1: a) This product was made according to the procedure of Example 1, with the exception that an equimolar mixture of 1-deoxy-1-(butylamino)-D-glucitol and 1-deoxy-1-(octylamino)-D-glucitol was used as the amine component in the linking reaction. The product consisted of an approximately 1:1:2 mixture of N,N'-dibutyl-N,N'-bis(1-deoxyglucityl) ethylenediamine, N,N'-dioctyl-N,N'-bis(1-deoxyglucityl)ethylenediamine, and N-butyl-N'-octyl-N,N'-bis(1-deoxyglucityl)ethylenediamine, respectively, on a mole ratio basis. The mixture of the three diamines was not separated, but was characterized and tested as prepared. | | | | | |

### Examples 9-13

These examples illustrate another aspect of this invention, the ability to use dinitriles as linking groups to prepare the gemini surfactants of this invention. This transformation is represented by the following equation:

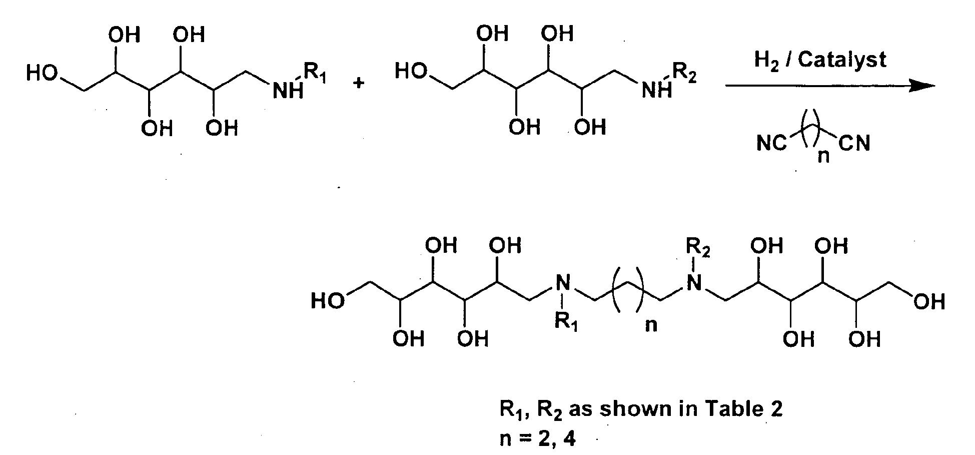

A 100-mL Parr stainless steel reactor was charged with 8.19 gm (0.042 mole; 2.1 equivalents) 1-deoxy-1-(methylamino)-D-glucitol, 2.16 gm of adiponitrile (0.02 mole; 1.0 equivalent), 0.52 gm (dry weight basis) 5% palladium on carbon, and 40 mL of 2-propanol. The reactor was closed, purged with nitrogen and hydrogen, and pressurized to ca 600 psig with hydrogen. The mixture was heated with stirring (1000 rpm) to 150°C and pressurized with hydrogen to 1250 psig. The reaction was maintained at this temperature; pressure was maintained at 1250 psig via regulated hydrogen feed. After 5 hr, the mixture was cooled to room temperature, and the product removed from the reactor by filtration through an internal 0.5µm sintered metal element. After trimethylsilylation, analysis of the product by GC and GC-MS indicated that it consisted of 62% N,N'-dimethyl-N,N'-bis(1-deoxyglucityl)hexamethylenediamine, 2% N-methyl-N-(5-cyanopentyl)-1-deoxyglucitylamine, 29% unchanged 1-deoxy-1-(octylamino)-D-glucitol, and minor amounts of byproducts. Additional N,N'-dialkyl-N,N'-bis(1-deoxyglucityl)-alkylenediamines were prepared and characterized using procedures similar to that above. Some of the N,N'-dialkyl-N,N'-bis(1-deoxyglucityl)alkylenediamines that were prepared according to this procedure and their designations are shown in Table 2.

**Table 2. N,N'-Dialkyl-N,N'-bis(1-deoxyglucityl)alkylenediamines via Coupling with Dinitriles**

| Example | NB Reference | Spacer Length (x)^{a} | R₁ | R₂ | Abbreviation |
|---|---|---|---|---|---|
| 9 | 19248-65 | 6 | CH₃ | CH₃ | DMDGHMDA |
| 10 | 19248-68 | 4 | n-C₄H₉ | n-C₄H₉ | DBDGBDA |
| 11 | 19466-46 | 4 | CH₃ | CH₃ | DMDGBDA |
| 12 | 19248-75 | 10 | CH₃ | CH₃ | DMDGDDA |
| 13 | 19248-76 | 8 | CH₃ | CH₃ | DMDGODA |

| | | | | | |
|---|---|---|---|---|---|
| Note to Table 2: a) The total spacer length, x, is two greater than n in the above equation. | | | | | |

### Examples 14-20

Equilibrium surface tensions were determined using a Kruss K-12 tensiometer with a platinum Wilhelmy plate, maintaining the temperature at 25 ± 1 °C by means of a constant temperature circulating bath. Results reported are averages of 10 measurements over a 10-minute period having a standard deviation of less than 0.01 mN/m. In many instances the solutions took hours to reach equilibrium. This equilibrium surface tension data along with that of the monoamine analogues (i.e. N-alkylglucamines) of compounds according to the invention are listed in Table 3.

**Table 3. Equilibrium Surfactant Data for N,N'-Dialkyl-N,N'-Bis(1-Deoxyglucityl)Alkylenediamines**

| EXAMPLE | SURFACTANT | EST (0.01 WT%, mN/m) | EST (0.1 WT%, mN/m) | EST (1.0 WT%, mN/m) |
|---|---|---|---|---|
| 14 | N-Butylglucamine | NA | NA | ≥ 50 |
| 15 | DBDGEDA | 62 | 50 | 47 |
| 16 | N-Hexylglucamine | 64 | 60 | 52 |
| 17 | DHDGEDA | 57 | 33 | 30 |
| 18 | N-Octylglucamine | 55 | 35 | 35 |
| 19 | DODGEDA | 30 | 27 | 27 |
| 20 | BODGEDA | 31 | 27 | 28 |

Some of the practical benefits of low equilibrium surface tension values are that less surfactant is required to reduce the surface tension of a formulation (enhancing its wetting properties) and less surfactant will be needed to stabilize emulsions. As seen in Table 3, compounds according to the invention demonstrate low equilibrium surface tension values, which may enhance the ability of formulations to wet a given surface.

### Examples 21-27

Surfactants of the invention also effectively reduce dynamic surface tension. Solutions in distilled and deionized water of the surfactants of the invention were prepared. Their dynamic surface tensions were measured using the maximum bubble pressure method as described in *Langmuir* **1986,** *2*, 428-432, and these data are provided in Table 4. These data provide information about the performance of a surfactant at conditions close to equilibrium (0.1 bubbles/sec) through high surface creation rates or dynamic conditions (10 bubbles/sec). In a practical sense, high surface creation rates refer to rapid processes such as a spray or roller-applied coating, a high speed printing operation, or the rapid application of an agricultural product or a cleaner.

**Table 4 Dynamic Surface Tension Data for N,N'-Dialkyl-N,N'-Bis(1-deoxyglucityl)alkylenediamines**

| EXAMPLE | SURFACTANT | DST (0.1 WT%, mN/m) 0.1b/s | DST (0.1 WT%, mN/m) 10b/s | DST (0.5 WT%, mN/m) 0.1b/s | DST (0.5 WT%, mN/m) 10b/s |
|---|---|---|---|---|---|
| 21 | N-Butylglucamine | 71 | 72 | 70 | 71 |
| 22 | DBDGEDA | 63 | 67 | 56 | 47 |
| 23 | N-Hexylglucamine | 68 | 70 | 55 | 57 |
| 24 | DHDGEDA | 37 | 51 | 30 | 32 |
| 25 | N-Octylglucamine | 54 | 65 | 48 | 62 |
| 26 | DODGEDA | 31 | 70 | 38 | 62 |
| 27 | BODGEDA | 38 | 64 | 28 | 36 |

The data of Table 4 indicate that a wide range of dynamic surface tension reduction values may be obtained with surfactant compounds according to the invention. Thus strong (Examples 24-26 at low bubble rates) and moderate to low (Example 22) surface tension reduction may be obtained. Depending upon the mode of application of a formulation and the substrate to be wetted (brush application of an industrial coating, spray application of an industrial cleaner, roller application of an adhesive), the range of surface tension reduction values provided by these surfactants covers a number of commercially useful applications.

### Examples 28-34

The foaming characteristics of the surfactants of Table 3 were evaluated using a slight modification of the Ross-Miles foam test (*Am. Soc. For Testing Materials,* Method D1173-53, Philadelphia, PA, **1953)** for solutions of 0.01 wt% and 0.1 wt% surfactant in water. Foam data for surfactants of the invention as well as monoamine analogues are shown in Table 5.

**Table 5 Foam Stability Data for N,N'-Dialkyl-N,N'-Bis(1-deoxyglucityl)alkylenediamines**

| EXAMPLE | SURFACTANT | Ross Miles Initial Foam (cm) | Ross Miles Final Foam (cm) |
|---|---|---|---|
| 28 | N-Butylglucamine | 2.5 | 0.3 |
| 29 | DBDGEDA | 1.8 | 0.5 |
| 30 | N-Hexylglucamine | 2.0 | 1.0 |
| 31 | DHDGEDA | 3.6 | 1.5 |
| 32 | N-Octylglucamine | 2.0 | 0.3 |
| 33 | DODGEDA | 5.2 | 4.8 |
| 34 | BODGEDA | 4.4 | 3.8 |

For the monoamine analogues of compounds according to the invention, a decrease or no change in initial foam height was observed with an increase in hydrophobe length (Examples 28, 30, and 32). In contrast, the surfactants of the invention showed an increase in initial foam height and foam stability with an increase in alkyl chain length (Examples 29, 31, 33, and 34). Thus a range of foam performance may be obtained, depending upon the alkyl group attached to the amine. While applications such as coatings, inks, and adhesives require low foam or foam that dissipates quickly, other applications such as cleaning, personal care, or ore floatation require a controlled amount of foam to be present and to persist. Therefore, the surfactants of the invention may be useful for a wide range of applications.

### Example 35

Comparison of the aqueous solubilities of several N,N'-dialkyl-N,N'-bis(1-deoxyglucityl)ethylenediamines was performed, with the results as follows:

| Composition | Compound(s) | Solubility (wt% in water) |
|---|---|---|
| 1 | DBDGEDA | >90 |
| 2 | DODGEDA | ≤0.02 |
| 3 | 1:1:2 DBDGEDA:DODGEDA:BODGEDA | 0.5 - >90 |
| 4 | 1:1 DBDGEDA:DODGEDA | </= 62 |

The ratios of compounds are on a molar basis. Although 1 is soluble and 3 is a mixture, the extreme insolubility of 2 argues that the mixture 3 should be poorly soluble. A control experiment (4) evaluated the solubility of a 1:1 molar mixture of 1 and 2 in water. Although this mixture had the same number of butyl and octyl groups as 3, it did not contain any N-butyl-N'-octyl-N,N'-bis(1-deoxyglucityl)ethylenediamine, and it was much less soluble in water: At 62 wt%, it dissolved with difficulty (only after > 1 hr at 85 - 90°C), and was insoluble at higher concentrations, while composition 3 was soluble up to about 0.25 wt% (not shown), gave a milky appearance between about 0.25 and 0.5 wt%, and was soluble thereafter to greater than a 90 wt% level. The high solubility of such a formulation may be helpful due to the ability to handle concentrated solutions of it, but it would be expected to have a higher surfactant efficiency than the other highly soluble composition (100% DBDGEDA). Thus a formulation requiring between about 5 and 15 wt% of composition 1 may require only about 1 wt% or possibly even less of composition 3 to achieve the same results.

Other compositions for which similar solubility behavior may occur also incorporate mixtures of shorter and longer (optionally functionalized) alkyl groups. Specific examples are shown by the following structure and table. Compositions employing surfactants wherein R₁ and R₂ are of different lengths L1 and L2 (and/or of different composition, e.g. different in functional group content) may incorporate mixtures having an approximately 1:1:2 ratio of compounds containing two L1 groups, two L2 groups, and one L1 and one L2 group, respectively. For example, L1 is butyl and L2 is octyl in composition 3 above. Thus they may overall have equimolar amounts of L1 and L2, distributed in such a way as to form a 1:1:2 mixture, with the amount of combined L1/L2 product therefore constituting 50 mol% of the mixture. Such a mixture typically results from using a reaction mixture containing equimolar amounts of the L1 and L2 groups. However, reaction mixtures richer in either L1 or L2 may be used, resulting in compositions having a higher proportion of that component and less than 50 mol% of molecules containing one each of L1 and L2. Such mixed compositions are also contemplated according to the invention, and may for example contain greater than 10 and less than 50 mol% of the combined L1/L2 molecules, more typically greater than 25 and less than 50 mol% of the combined L1/L2 molecules.

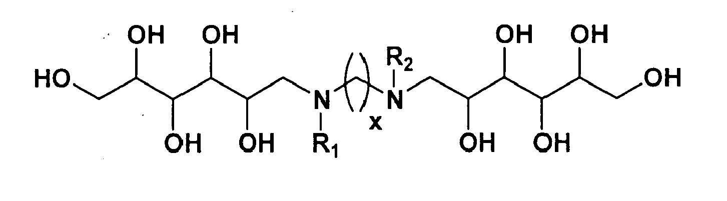

| R₁ | R₂ | x |
|---|---|---|
| 1-butyl | 1-octyl | 4, 6 |
| methyl or ethyl | 1-hexyl or 1-octyl | 2-6 |
| (CH₂)₃OCH₃ | 1-hexyl or 1-octyl | 2-6 |
| (CH₂)₂OCH₃ | 1-hexyl or 1-octyl | 2-6 |

### Examples 36-41

Although dynamic and equilibrium surface tension as well as foam influence the cleaning ability of a surfactant, other factors also affect cleaning performance. Thus one good way to determine the cleaning ability of a surfactant is to apply a soil to a substrate, clean that substrate with the surfactant of interest and some benchmark surfactants, and compare relative cleaning efficacies in terms of percent soil removal. One method for conducting such testing is described in ASTM D4488-95, "Standard Guide for Testing Cleaning Performance of Products Intended for Use on Resilient Flooring and Washable Walls." This method was followed according to the particulate and oily soil/vinyl tiles test methodology, with cleaning performance being evaluated by reading average reflectance values (5 readings/tile) of a white vinyl composition tile before and after cleaning (Table 6). The higher the reflectance value the better the cleaning ability of the surfactant.

The cleaning performance of surfactants according to the invention was compared side by side with that of two benchmark surfactants, Neodol 23-6.5 (available from Shell Chemical of Houston, Texas) and AG 6202 (available from Akzo Nobel of McCook, Illinois), an alcohol ethoxylate and an alkylpolyglucoside surfactant, respectively. Comparisons with blank samples (no surfactant) were also run to demonstrate the effect of sponge abrasion without the presence of surfactant.

**Table 6 Cleaning Performance of N,N'-Dialkyl-N,N'-bis(1-deoxyglucityl)alkylenediamines**

| EXAMPLE | SURFACTANT | CONCENTRATION (wt%) | % SOIL REMOVAL |
|---|---|---|---|
| 36 | Neodol 23-6.5 | 0.1 % | 47 |
| 37 | AG 6202 | 0.1% | 42 |
| 38 | DBDGEDA | 0.1 % | 45 |
| 39 | DHDGEDA | 0.1% | 67 |
| 40 | DODGEDA | 0.1% | 48 |
| 41 | None | NA | 18 |

As noted in Table 6, all surfactants of the invention demonstrated better cleaning performance via higher % soil removal than the benchmark alkylpolyglucoside AG 6202 with some surfactants of the invention also exceeding the cleaning performance of the benchmark product, Neodol 23-6.5.

Surfactants according to the invention may find significant utility as emulsifiers, wetting agents, foaming agents, defoamers, rheology modifiers or associative thickeners, dispersants, and the like, and especially as detergents. As such, these compounds are useful in applications such as coatings, inks, adhesives, agricultural formulations, fountain solutions, photoresist strippers/developers, templating agents for mesoporous materials, soaps, shampoos, hard surface cleaning, other cleaning compositions, and in cement admixture formulations. The compounds should also find use in oil-field applications such as enhanced oil recovery, fracturing and stimulation processes, drilling and cementing operations, and in various wet-processing textile operations, such as the dyeing of fibers and fiber scouring and kier boiling.

Although the invention is illustrated and described herein with reference to specific embodiments, it is not intended that the subjoined claims be limited to the details shown. Rather, it is expected that various modifications may be made in these details by those skilled in the art, which modifications may still be within the spirit and scope of the claimed subject matter and it is intended that these claims be construed accordingly.

## Claims

1. A composition comprising at least one compound according to structure (I): 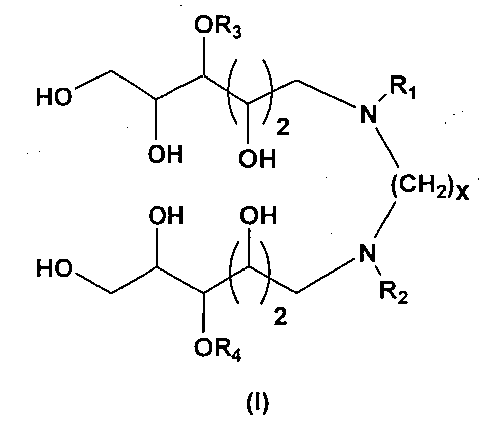 wherein x is an integer from 1 to 12; R₁ is 1-octyl or 1-butyl and R₂ is 1-octyl or 1-butyl; and R₃ and R₄ are independently selected from the group consisting of hydrogen, α-D-glucopyranosyl, β-D-glucopyranosyl, and β-D-galactopyranosyl.

2. The composition of claim 1, wherein the at least one compound comprises:
a) a first compound wherein R₁ is 1-butyl and R₂ is 1-octyl;
b) a second compound wherein R₁ and R₂ are both 1-octyl; the composition further comprising:
c) a third compound according to structure (I) wherein R₁ and R₂ are both 1-butyl; wherein the second, third, and first compounds are in a molar ratio of about 1:1:2, respectively.

3. The composition of claim 1, wherein x is an integer from 2 to 4.

4. The composition of claim 1, wherein the at least one compound is N,N'-dioctyl-N,N'-bis(1-deoxyglucityl)-1,2-diaminoethane.

5. The composition of claim 1, wherein the at least one compound is N-butyl-N'-octyl-N,N'-bis(1-deoxyglucityl)-1,2-diaminoethane.

6. A composition comprising at least one compound according to structure (I): 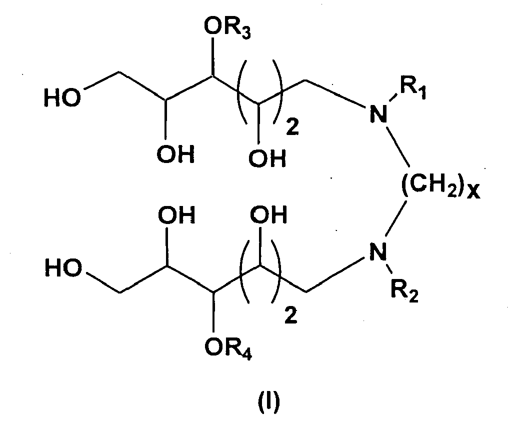 wherein x is an integer from 2 to 6; R₁ is methyl or ethyl and R₂ is 1-hexyl or 1-octyl; and R₃ and R₄ are both H.

7. A composition comprising at least one compound according to structure (I): 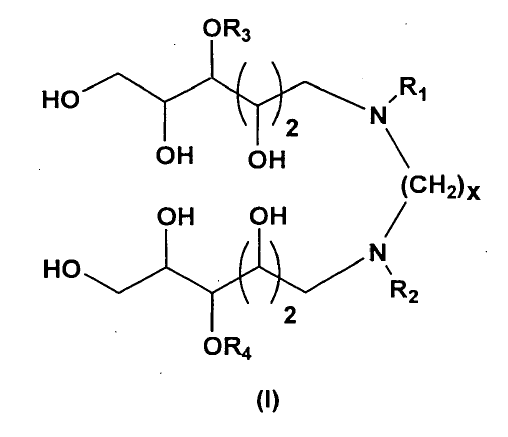 wherein x is an integer from 2 to 6; R₁ is (CH₂)₂OCH₃ or (CH₂)₃OCH₃ and R₂ is 1-hexyl or 1-octyl; and R₃ and R₄ are both H.

8. A method of making a compound according to structure (I): 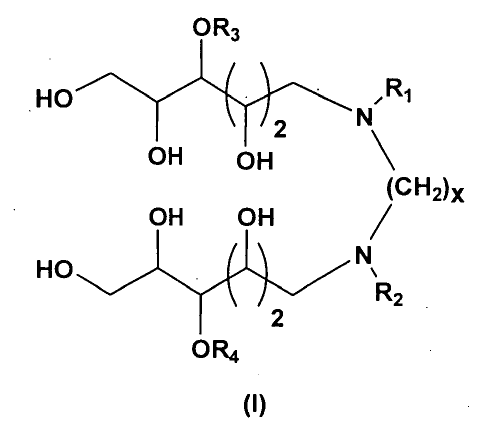 wherein x is an integer from 1 to 12; R₁ and R₂ are independently selected from the group consisting of C3 - C30 linear alkyl, cyclic alkyl, branched alkyl, alkenyl, aryl, alkylaryl, alkoxyalkyl, and dialkylaminoalkyl; and R₃ and R₄ are independently selected from the group consisting of hydrogen, α-D-glucopyranosyl, β-D-glucopyranosyl, and β-D-galactopyranosyl;
the method comprising contacting an N-(polyhydroxyalkyl)alkylamine with a dinitrile, a dialdehyde, or an acetal or hemiacetal thereof, said contacting performed in the presence of hydrogen and a transition metal catalyst.

9. The method of claim 8, wherein the dialdehyde or acetal or hemiacetal thereof is a dialdehyde.

10. The method of claim 8, wherein R₁ is 1-octyl or 1-butyl and R₂ is 1-octyl or 1-butyl.

11. The method of claim 8, wherein x is an integer from 2 to 4.

12. The method of claim 10, wherein x is 2 and R₃ and R₄ are both hydrogen.

13. The method of claim 8, wherein the compound is N,N'-dibutyl-N,N'-bis(1-deoxyglucityl)-1,2-diaminoethane, N,N'-dioctyl-N,N'-bis(1-deoxyglucityl)-1,2-diaminoethane, N-butyl-N'-octyl-N,N'-bis(1-deoxyglucityl)-1,2-diaminoethane, or N,N'-dihexyl-N,N'-bis(1-deoxyglucityl)-1,2-diaminoethane.

14. The method of claim 8, wherein the catalyst is selected from the group consisting of iron, cobalt, nickel, ruthenium, rhodium, palladium, osmium, iridium and platinum, preferably Ru, Rh, Pd and Pt.

15. The method of claim 8, wherein the catalyst is supported on carbon.

16. The method of claim 8, wherein the contacting is performed at a temperature between about 50°C and about 175°C.

17. The method of claim 8, wherein the contacting is performed in the presence of a solvent.

18. The method of claim 17, wherein the solvent comprises methanol, ethanol, ethylene glycol, propylene glycol, a water/methanol mixture, a water/ethanol mixture, or a combination of any of these, preferably a water/methanol mixture.

19. A hard surface cleaning formulation comprising water and between 0.1 and 99 wt% in total of one or more ingredients selected from the group consisting of anionic surfactants, cationic surfactants, nonionic surfactants other than N,N'-dialkyl-N,N'-bis(polyhydroxyalkyl)alkylenediamines, solvents, alkali metal hydroxides, and between 0.001 and 25 wt% of the composition of claim 1.

20. A coating formulation comprising 5 and 99.9 wt% of a water-borne, water-dispersible, or water-soluble resin, between 0.01 and 10 wt% in total of one or more other additives selected from the group consisting of surfactants, wetting agents, and flow and leveling agents, other than N,N'-dialkyl-N,N'-bis(polyhydroxyalkyl)alkylenediamines, and between 0.001 and 5 wt% of the composition of claim 1.

21. An ink formulation comprising 1 and 50 wt% of a pigment, 5 and 99.9 wt% of a water-borne, water-dispersible, or water-soluble resin, 0.01 and 10 wt% of a surfactant or wetting agent other than an N,N'-dialkyl-N,N'-bis(polyhydroxyalkyl)alkylenediamine, between 0.01 and 10 wt% in total of one or more other additives selected from the group consisting of processing aids, defoamers, and solubilizing agents, between 0.001 and 5 wt% of the composition of claim 1.

22. An agricultural formulation comprising between 0.1 and 50 wt% of a pesticide or plant growth modifying agent, between 0.01 to 10 wt% of a surfactant or wetting agent other than an N,N'-dialkyl-N,N'-bis(polyhydroxyalkyl)alkylenediamine, the improvement and between 0.001 and 5 wt% of the composition of claim 1.

23. A fountain solution formulation for planographic printing comprising between 0.05 and 10 wt% of a water-soluble, film forming macromolecule, between 1 and 25 wt% of a water-soluble alcohol, glycol, or polyol, between 0.01 and 20 wt% of a water-soluble acid or its salt, between 30 and 98.939 wt% of water, and between 0.001 and 5 wt% of the composition of claim 1.

24. A photoresist developer formulation comprising between 0.1 and 3 wt% of tetramethylammonium hydroxide, between 92.5 and 99.9 wt% of water, and between 0.001 and 5 wt% of the composition of claim 1.

25. A synthetic metalworking fluid formulation comprising between 2.5 and 10 wt% of an emulsifying agent, between 10 and 25 wt% of an alkanolamine, between 2 and 10 wt% of an organic monoacid, between 1 and 5 wt% of a biocide, between 40 and 84.499 wt% of water, and between 0.001 and 5 wt% of the composition of claim 1.

26. A rinse aid formulation comprising water between 5 and 20 wt% of a chelant, and between 0.001 and 45 wt% of the composition of claim 1.

27. A powdered laundry detergent formulation comprising between 0.1 and 50 wt% of one or more detergent surfactants, between 25 and 60 wt% of a builder or co-builder, and between 0.001 and 15 wt% of the composition of claim 1.

28. An aqueous liquid laundry detergent formulation comprising between 0.1 and 65 wt% of one or more detergent surfactants, between 3 and 36 wt% of a builder or co-builder, 0.1 and 5 wt% in total of one or more other additives selected from the group consisting of fragrances and dyes, between 1 and 75 wt% in total of one or more other additives selected from the group consisting of water and other solvents, and between 0.001 and 30 wt% of the composition of claim 1.

29. A non-aqueous laundry detergent formulation comprising between 0.1 and 42 wt% of one or more detergent surfactants, 25 and 60 wt% of a builder or co-builder, between 0.5 and 5 wt% of an anti-redeposition aid, and between 0.001 and 30 wt% of the composition of claim 1.

30. An industrial and institutional laundry detergent formulation comprising water and between 0.01 and 2 wt% of an anti-redeposition aid, and 0.001 and 20 wt% of the composition of claim 1.

31. A shampoo or liquid body wash formulation comprising water between 0.1 and 30 wt% of an anionic surfactant and between 0.001 and 5 wt% of the composition of claim 1.

32. A hair conditioner formulation comprising water between 0.1 and 10 wt% of a nonionic surfactant other than an N,N'-dialkyl-N,N'-bis(polyhydroxyalkyl)alkylenediamine, and between 0.001 and 10 wt% of the composition of claim 1.

33. An aqueous sunscreen formulation comprising water between 1 and 30 wt% of a sunscreen agent, and between 0.001 to 30 wt% of the composition of claim 1.

34. A cement admixture formulation comprising between 40 and 75 wt% of water between 0.1 and 20 wt% in total of one or more solubilizing agents, polymers, oligomers, or functional additives, and between 0.001 and 5 wt% of the composition of claim 1.

35. Use of the compound according to one of claims 1 to 7 or a mixture of such compounds in a formulation selected from the group consisting of a hard surface cleaning formulation, a coating formulation, an ink formulation, an agricultural formulation, a fountain solution formulation, a photoresist developer formulation, a synthetic metalworking fluid formulation, a rinse aid formulation, a powdered laundry detergent formulation, an aqueous liquid laundry detergent formulation, a non-aqueous laundry detergent formulation, an industrial and institutional laundry detergent formulation, a shampoo or liquid body wash formulation, a hair conditioner formulation, an aqueous sunscreen formulation and a cement admixture formulation.
